# EUROPEAN PATENT SPECIFICATION

(11) **EP 3 255 960 B1**
(45) Date of publication and mention of the grant of the patent: **27.10.2021**
(21) Application number: 16746881.8
(22) Date of filing: 05.02.2016
(51) Int. Cl.: H05H 1/24

(54) **ANIONS GENERATOR COMPRISING A PLASMA SOURCE COMPRISING POROUS DIELECTRIC**
ANIONENGENERATOR MIT PLASMAQUELLE MIT PORÖSEM DIELEKTRIKUM
GÉNÉRATEUR D'ANIONS COMPRENANT UNE SOURCE DE PLASMA COMPRENANT UN DIÉLECTRIQUE POREUX

(30) Priority: 05.02.2015 KR 20150017875
(43) Date of publication of application: 13.12.2017
(60) Divisional application: 21197401.9
(73) Proprietor: Korea Institute of Fusion Energy, Daejeon 34133 (KR)
(72) Inventor: SEOK, Dong Chan, Gunsan-si Jeollabuk-do 54087 (KR); JUNG, Yong Ho, Seoul 05058 (KR); CHOI, Yong Sup, Gunsan-si Jeollabuk-do 54087 (KR); LHO, Taihyeop, Seoul 08269 (KR); LEE, Kang Il, Gunsan-si Jeollabuk-do 54099 (KR); JEONG, Hyun Young, Gunsan-si Jeollabuk-do 54044 (KR)
(74) Representative: Laine IP Oy
(86) International application number: PCT/KR2016/001311
(87) International publication number: WO 2016/126140

(56) References cited:
- JP-A- 2009 054 557
- JP-A- 2010 177 002
- JP-A- 2012 011 301
- JP-A- 2012 011 301
- JP-A- 2013 211 204
- JP-A- 2013 211 204
- KR-B1- 101 256 577
- US-A1- 2011 101 862
- US-A1- 2013 333 841

## Description

### FIELD

The present invention relates to a plasma-generating source including a porous dielectric member partially immersed in liquid into a plasma generation region between two opposite electrodes for generating dielectric barrier discharge plasma, such that generation of ozone and nitrogen oxides is suppressed while generation of hydroxy radicals is promoted.

### BACKGROUND

A conventional anion generator using a plasma-generating source employs a corona discharge approach using asymmetric electrodes. In the corona discharge using the asymmetric electrodes, the corona discharge is generated by applying a voltage to a needle-shaped or wire electrode spaced apart from a plate-type electrode. This is a method of generating a corona plasma of very weak intensity by applying a high voltage to the asymmetric electrodes using a power supply to supply a high voltage having a very high impedance. In this connection, when a very thin wire or a needle-shaped metal electrode is present in an electric field space, the electric field is concentrated on a sharp end, and, thus, even a small voltage leads to dielectric breakdown (generation of plasma) of gas. As the plasma generated at the sharp end expands to a counter electrode, the intensity of the electric field rapidly decreases. Thus, this suppress a transition to spark or arc discharge due to thermal electron emission resulting from excessive current channel on an electrode surface is limited. Rather, the electric field weakens in all directions around the electrode, so that a radial discharge shape appears around the electrode.

However, when liquid mist, dust, or the like is present between the electrodes, spark and arc discharge may occur, thereby causing the surface of the electrode to corrode or to causing the dust to be accumulated on the electrode surface, which deteriorates performance over a long period of use. In addition, since the volume of the generated plasma is small and the plasma intensity is weak, anion generating efficiency is relatively low. In addition, a large amount of ozone and nitrogen oxides (NOx) may be generated, thereby to generate unpleasant odor and/or to be harmful to the human body. In addition, electrode corrosion occurs at the time of long-term use, and the anion and the active species required for sterilization may have a short active duration and may not be suitable for a wide space application.

In order to overcome the disadvantages of the corona discharge method using such asymmetric electrodes, a dielectric barrier discharge plasma method is used. In this method, one or more dielectrics are inserted between two metal electrodes to generate a uniform volume of plasma, which is used as a sterilizing agent.

By using a configuration in which one or more dielectrics are interposed between the two metal electrodes, a flat plate-like or cylindrical-type configuration may be possible. An electric field is applied between the two electrodes to cause dielectric breakdown (plasma generation) of gas in a space between the electrodes.

Generally, in case of non-RF or non-micro wave, when dielectric breakdown of gas occurs in a space between metals, a plasma channel having a weak current density such as a streamer is first formed. Thereafter, when the electric field is maintained for a certain period, heat is generated on the surface of the metal electrode in contact with the plasma channel, and, thus, an enormous amount of hot electrons are emitted from the metal surface, and eventually, the plasma channel is transitioned to arc or spark discharge (small plasma volume, electrode damage). In the dielectric barrier discharge plasma method, the formation of the plasma channel having the excessive current density as described above is limited by inserting a non-conductive dielectric between the metal electrodes, thereby suppressing the transition to the spark and arc discharge. However, in this manner as well, a large amount of ozone and nitrogen oxides (NOx) may be generated, which causes an unpleasant odor and is harmful to the human body. Likewise, the activation time of anions and active species required for sterilization is short, which is not suitable for application in a wide space. In addition, the sterilization effect is greatly influenced by the relative humidity in the atmosphere.

JP2012011301 A discloses an apparatus for treating water with plasma generated between a dielectric layer and a porous insulator.

JP 2013211204 A discloses a submerged apparatus for purifying liquid by means of plasma generated in a chamber which houses a porous dielectric.

US 2013/333841 A1 discloses an underwater electrical discharge container with a porous partition wall between a gas container and a liquid container. The apparatus comprises an electrode arranged in the gas container and another electrode arranged in the liquid container.

US 2011/101862 A1 discloses an apparatus for generating and directing chemically reactive plasma-generated species in a plasma device with a two coaxially arranged electrodes.

### SUMMARY

The present invention is to provide a plasma-generating source including a porous dielectric member partially immersed in liquid into a plasma generation region between two opposite electrodes for generating dielectric barrier discharge plasma, such that generation of ozone and nitrogen oxides is suppressed while generation of hydroxy radicals is promoted.

The present invention discloses an anion generator according to claim 1. Additional preferred aspects are disclosed in the dependent claims.

### ADVANTAGEOUS EFFECTS

In the anions generator comprising the plasma-generating apparatus according to the present invention, porous or fibrous dielectric member may absorb liquids. Thus, this leads to a high combined dielectric constant of water and dielectrics, thereby creating a high electric field around these dielectrics. The moisture content in the air greatly influences the generation of negative ions, and in the case of dry weather, negative ion generation is reduced. However, according to a dependent claim, water is supplied into the porous dielectric which in turn supplies the moisture to the electrode, and plasma is directly reacted with water in the liquid or vapor state, so that the efficiency of generating negative ions can be maximized. Particularly, ozone and nitrogen oxides generated during the electric discharge are discharged as they are, and they may be harmful to the human body while staying in the indoor space for a relatively long period of time. However, in accordance with the present invention, ozone, or nitrogen oxides may be absorbed into water impregnated into the porous dielectric member, or may be converted to OH radicals with combining with vapors generated from the electrodes. Thus, the metal electrode is not exposed to the discharge space, and, thus, there is no transition to the spark or arc discharge. Further, there is no metal corrosion. Metal foreign substance resulting from sputtering or the like may not be discharged into the air.

### DESCRIPTION OF DRAWINGS

FIG. 1 is a schematic diagram of a dielectric barrier discharge plasma-generating source containing a porous dielectric member partially immersed in liquid, according to one embodiment of the present invention.
FIG. 2 is a schematic diagram illustrating the generation of a large amount of OH radicals by the plasma-generating source in accordance with the present invention.
FIG. 3 to FIG. 8 are schematic diagrams of dielectric barrier discharge plasma-generating sources, each source containing a porous dielectric member partially immersed in liquid, according to further embodiments of the present invention.
FIG. 9 is a schematic diagram illustrating a plasma-generating source according to the present invention applied as a deposition apparatus.

### DETAILED DESCRIPTION

Examples of various embodiments are illustrated and described further below. It will be understood that the description herein is not intended to limit the claims to the specific embodiments described. On the contrary, it is intended to cover alternatives, modifications, and equivalents as may be included within the scope of the present invention as defined by the appended claims.

It will be understood that, although the terms "first", "second", "third", and so on may be used herein to describe various elements, components, regions, layers and/or sections, these elements, components, regions, layers and/or sections should not be limited by these terms. These terms are used to distinguish one element, component, region, layer or section from another element, component, region, layer or section. Thus, a first element, component, region, layer or section described below could be termed a second element, component, region, layer or section, without departing from the scope of the present disclosure.

The terminology used herein is for the purpose of describing particular embodiments only and is not intended to be limiting of the present disclosure. As used herein, the singular forms "a" and "an" are intended to include the plural forms as well, unless the context clearly indicates otherwise. It will be further understood that the terms "comprises", "comprising", "includes", and "including" when used in this specification, specify the presence of the stated features, integers, operations, elements, and/or components, but do not preclude the presence or addition of one or more other features, integers, operations, elements, components, and/or portions thereof. As used herein, the term "and/or" includes any and all combinations of one or more of the associated listed items. Expression such as "at least one of' when preceding a list of elements may modify the entire list of elements and may not modify the individual elements of the list.

Unless otherwise defined, all terms including technical and scientific terms used herein have the same meaning as commonly understood by one of ordinary skill in the art to which this inventive concept belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

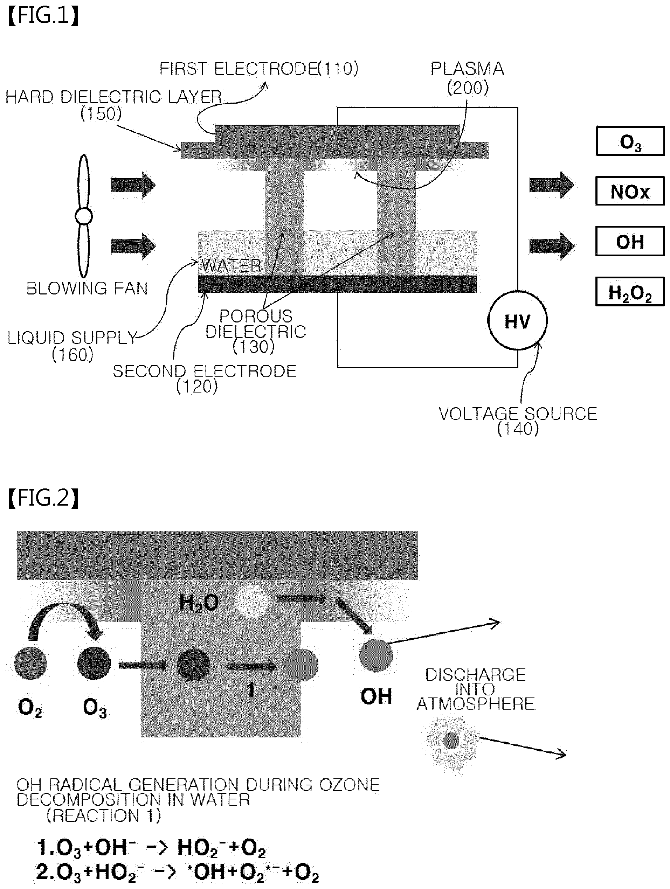

FIG. 1 is a schematic diagram of a dielectric barrier discharge plasma-generating source containing porous dielectric member partially immersed in liquid, according to one embodiment of the present invention. In particular, an anion generator including the plasma-generating source according to one embodiment of the present invention is exemplified.

The plasma-generating source according to one embodiment of the present invention includes: a plate-like first electrode 110; a plate-like second electrode 120 facing away from the first electrode 110; at least one porous dielectric member disposed between the first and second electrodes 110 and 120; a voltage source 140 for applying an AC voltage to the first electrode and the second electrode 110 and 120; a dielectric layer 150 formed on one face of at least one of the first electrode and the second electrode 110 and 120; and a liquid supply 160 containing liquid therein. One end of the porous dielectric member 130 is immersed in the liquid within the liquid supply 160, thereby supplying the liquid to the porous dielectric member 130.

When an AC voltage is applied to the first electrode and the second electrode 110 and 120 by the voltage source 140, a plasma 200 is generated between the first electrode and the second electrode 110 and 120. At least one porous dielectric members 130 may be located in the plasma 200 generation region. As a result, the plasma is placed in a highly humid environment. As a result, as shown in FIG. 2, a large amount of OH radicals are generated and a large amount of negative ions are generated. A blowing fan may be provided to release the generated negative ions.

The liquid may comprise water. High polarity liquids such as water have a high dielectric constant (about 80). In addition, alumina or glass material has a dielectric constant of 10 and 5, respectively. Porous ceramics having water impregnated therein may have a high dielectric constant of 40 to 45. Therefore, when the porous ceramic having water impregnated therein is close to the counter electrode, a high electric field may be applied to a position adjacent to the porous ceramic, thereby realizing a structure that easily generates plasma.

FIG. 2 is a schematic diagram illustrating a state where the plasma-generating source according to an embodiment of the present invention generates a large amount of OH radicals. Ozone generated by the plasma reacts with H₂O impregnated in the porous dielectric member to produce a large amount of OH radicals. Further, due to collisions of ions generated by the plasma or heat resulting from the collisions, the liquid forms liquid mist or clusters, which, in turn, may be discharged to the atmosphere. At this time, active species having a short survival period may be adsorbed to the liquid cluster which in turn diffuses into the atmosphere. In this connection, the clusters act as a protective film, and, thus, the lifetime of the active species can be kept long.

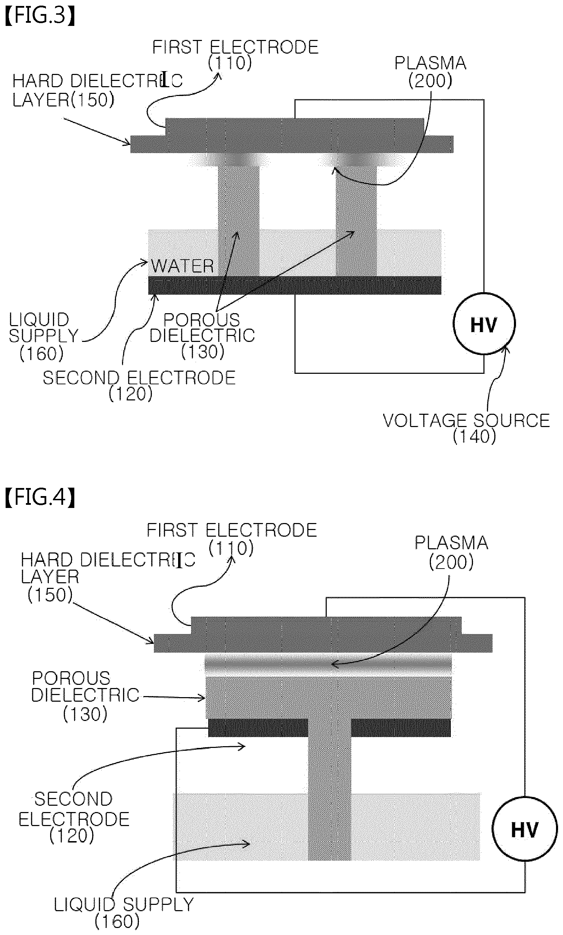

FIG. 3 to FIG. 8 are schematic diagrams of dielectric barrier discharge plasma-generating sources, each source containing a porous dielectric member partially immersed in liquid, according to further embodiments of the present invention.

In particular, FIG. 3 illustrates a planar dielectric barrier discharge plasma-generating apparatus. In this embodiment, the porous dielectric member 130 is oriented perpendicular to the horizontally-extending first and second electrodes 110 and 120. The other end of the at least one porous dielectric member 130 is spaced apart from the dielectric layer 150.

FIG. 4 illustrates a further planar dielectric barrier discharge plasma-generating apparatus. In this embodiment, the porous dielectric member 130 have vertical and horizontal portions. The vertical portion is oriented perpendicular to the horizontally-extending first and second electrodes 110 and 120. The horizontal portion of the member 130 is parallel to the horizontally-extending first and second electrodes 110 and 120. One end of the vertical portion of the member 130 is partially immersed in the liquid in the liquid supply 160. The horizontal portion of the porous dielectric member 130 is spaced apart from the dielectric layer 150.

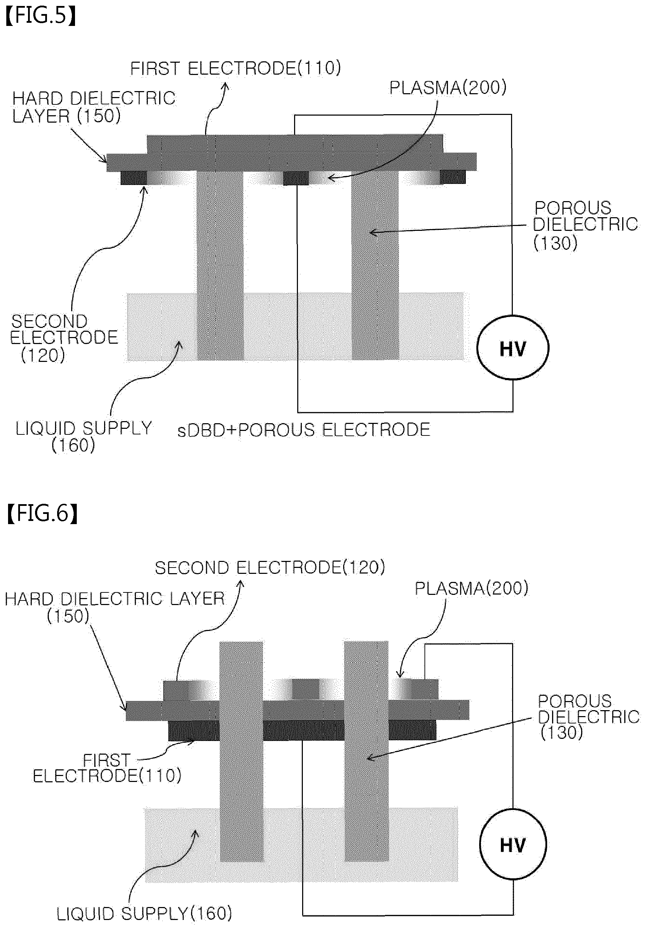

FIG. 5 illustrates a surface DBD (dielectric barrier discharge) plasma-generating apparatus (sDBD plasma-generating apparatus). In this embodiment, the apparatus includes a plate-like dielectric layer 150; a plate-like first electrode 110 disposed on a first face of the dielectric layer; a second electrode array 120 disposed on a second face of the dielectric layer 150 opposite the first face. The second electrode array 120 may be configured such that a plurality of electrode bars are arranged in parallel with each other or dot-like electrodes are arranged spacedly apart from each other in a matrix form. At least one porous dielectric member 130 is disposed between the electrode bars or the dot-like electrodes of the second electrode array 120. When a voltage is applied to the first electrode and the second electrode array by the voltage source HV, a plasma 200 is generated between the first electrode and the second electrode array. One end of at least one porous dielectric member 130 may be located in the plasma generation region.

FIG. 6 illustrates a further surface DBD (dielectric barrier discharge) plasma-generating apparatus (sDBD plasma-generating apparatus). In this embodiment, the apparatus includes a plate-like dielectric layer 150; a plate-like first electrode 110 disposed on a first face of the dielectric layer 150; a second electrode array 120 disposed on a second face of the dielectric layer 150 opposite the first face. The second electrode array 120 may be configured such that a plurality of electrode bars are arranged in parallel with each other or dot-like electrodes are arranged spacedly apart from each other in a matrix form. At least one porous dielectric member 130 passes through the plate-like dielectric layer 150 and between the electrode bars or the dot-like electrodes of the second electrode array 120. When a voltage is applied to the first electrode and the second electrode array by the voltage source HV, a plasma 200 is generated between the first electrode and the second electrode array. At least one porous dielectric member 130 may be partially located in the plasma generation region.

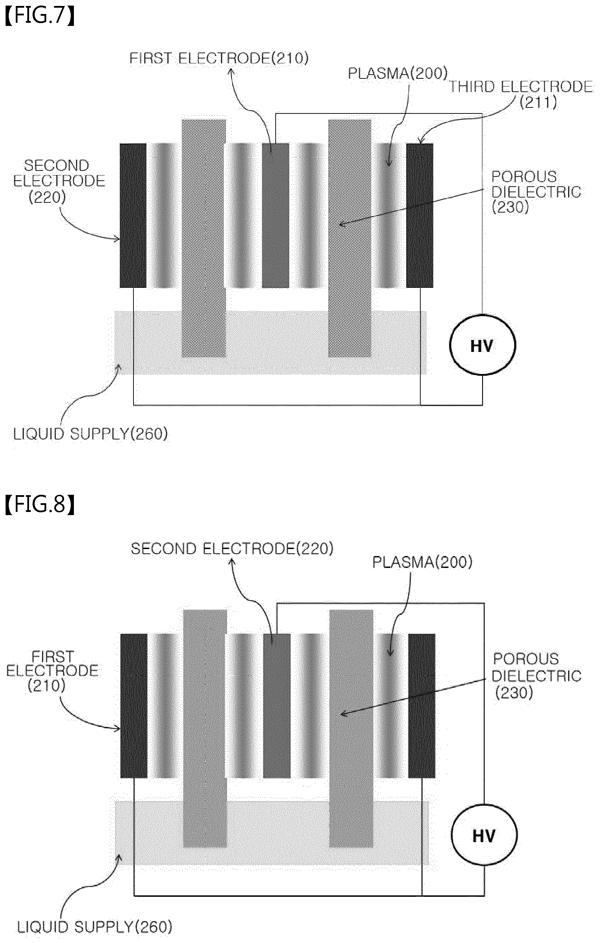

FIG. 7 illustrates a stack-type DBD (dielectric barrier discharge) plasma-generating apparatus. In this embodiment, the apparatus includes a vertically oriented plate-like first electrode layer 210; a vertically oriented plate-shaped second electrode layer 220 spaced from and parallel to the first electrode layer; and a vertically oriented plate-shaped third electrode layer 211 spaced apart from and in parallel with the second electrode layer, wherein the second electrode layer is disposed between the first electrode layer and the third electrode layer. The apparatus includes first and second porous dielectric members 230 disposed respectively between the first electrode layer 210 and the second electrode layer 220 and between the second electrode layer 220 and the third electrode layer 211. The first porous dielectric member 230 is spaced from each of the first electrode layer 210 and the second electrode layer 220. The second porous dielectric member 230 is separated from each of the second electrode layer 220 and the third electrode layer 211. When a voltage is applied between the first electrode layer 210 and the second electrode layer 220 and between the third electrode layer 211 and the second electrode layer 220 by the voltage source HV, plasmas may be generated between the electrodes. In this case, the first and second porous dielectric members 230 may be partially located in the plasma generation region.

FIG. 8 illustrates a cylindrical DBD (dielectric barrier discharge) plasma-generating apparatus. In this embodiment, the apparatus includes a hollow cylindrical first electrode 210; a vertical bar-like second electrode 220 spaced from the first electrode, wherein the second electrode is disposed at a central position in the hollow cylindrical first electrode 210. The apparatus includes a hollow cylindrical porous dielectric member 230 disposed between the first and second electrodes. The hollow cylindrical porous dielectric member 230 may be spaced from the first and second electrodes. The hollow cylindrical porous dielectric member 230 surrounds the vertical bar-like second electrode 220. When a voltage is applied between the first electrode 210 and the second electrode 220 by the voltage source HV, a plasma may be generated between the electrodes. In this case, the dielectric member 230 may be partially located in the plasma generation region.

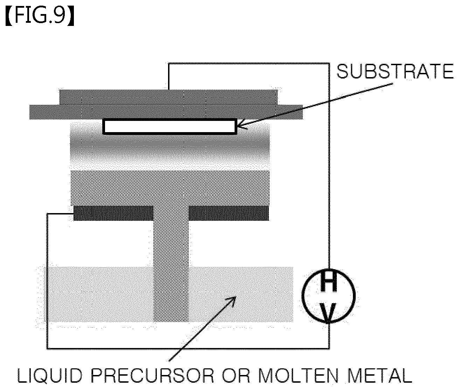

FIG. 9 is a schematic diagram illustrating a plasma-generating source according to the present invention applied as a deposition apparatus. The deposition apparatus a plate-like first electrode; a plate-like second electrode facing away from the first electrode; and a porous dielectric member have vertical and horizontal portions. The vertical portion is oriented perpendicular to the horizontally-extending first and second electrodes and. The horizontal portion of the member is parallel to the horizontally-extending first and second electrodes. One end of the vertical portion of the member is partially immersed in liquid precursor to be deposited or melted metal in in the liquid supply. The horizontal portion of the porous dielectric member is spaced apart from the dielectric layer. A substrate is disposed on the dielectric layer. The porous dielectric member impregnated with the molten metal or liquid precursor may react with the generated plasma, and, then, may be evaporated or atomized. The evaporated or atomized precursor may be deposited on a substrate. The use of the present apparatus can save consumption of material and have application for a larger area deposition over a conventional solid-phase deposition target. In addition, using the present apparatus, the deposition rate can also be increased since the molten metal or liquid precursor has a lower binding energy than that of the solid-phase deposition target. Further, it is possible to deposit an organic film even under the atmospheric pressure and low temperature condition.

The description of the disclosed embodiments has been provided to enable any person skilled in the art to make or use the invention. Various modifications to these embodiments will be readily apparent to those skilled in the art. The scope of the invention is defined by the claims.

## Claims

1. An anion generator comprising a plasma generating apparatus, the plasma generating apparatus comprising:
- a first electrode (110);
- a second electrode (120) spaced apart from the first electrode (110);
- at least one porous dielectric member (130) disposed between the first and second electrodes (110, 120),
- a dielectric layer (150) formed on a first face of the first electrode (110) and/or the second electrode (120); and
- a voltage source (140) configured to apply an alternating voltage to and between the first electrode (110) and the second electrode (120), wherein when a voltage is applied to and between the first electrode (110) and the second electrode (120) using the voltage source (140), dielectric barrier discharge plasma (200) is generated between the first electrode (110) and the second electrode (120),
**characterized in that**:
- the at least one porous dielectric member (130) is partially immersed in liquid, and **in that**
- the at least one porous dielectric member (130) is partially located in the plasma-generation region.

2. The plasma generating apparatus of the anion generator of claim 1, further comprising a liquid supply (160) containing therein the liquid, wherein one end of the porous dielectric member (130) is immersed in the liquid in the liquid supply (160) such that the porous dielectric member (130) is impregnated with the liquid.

3. The plasma generating apparatus of the anion generator of claim 1, wherein each of the first and second electrodes (110, 120) horizontally extends, wherein the porous dielectric member (130) is oriented perpendicular to the first and second electrodes (110, 120), wherein a first end of the porous dielectric member (130) contacts the dielectric layer (150), wherein the first end of the porous dielectric member is partially located in the plasma-generation region.

4. The plasma generating apparatus of the anion generator of claim 1, wherein each of the first and second electrodes (110, 120) horizontally extends, wherein the porous dielectric member (130) is oriented perpendicular to the first and second electrodes (110, 120), wherein a first end of the porous dielectric member (130) faces away the dielectric layer (150), wherein the first end of the porous dielectric member is partially located in the plasma-generation region.

5. The plasma generating apparatus of the anion generator according to any one of the preceding claims, wherein:
- the first and second electrodes (110, 120) are plate-like and wherein
- the second electrode (120) faces away from the first electrode (110).

6. The plasma generating apparatus of the anion generator of claim 5, wherein:
- the plate-like first electrode (110) is an electrode layer (210);
- the plate-like second electrode (120) is an electrode layer (220) which is spaced from and parallel to the first electrode layer (210);
- the plasma a generating apparatus of the anion generator comprises a plate-shaped third electrode layer (211) spaced apart from and in parallel with the second electrode layer (220), wherein the second electrode layer (2 2 0) is disposed between the first electrode layer (210) and the third electrode layer;
- the at least one porous dielectric member comprises a first and second porous dielectric members (230) disposed respectively between the first electrode layer (210) and the second electrode layer (220) and between the second electrode layer (220) and the third electrode layer, wherein the first porous dielectric member (230) is spaced from each of the first electrode layer (210) and the second electrode layer (220), wherein the second porous dielectric member (230) is spaced from each of the second electrode layer (220) and the third electrode layer,
- the voltage source is configured to apply a voltage to and between the first electrode layer (210) and the second electrode layer (220), and to and between the first electrode layer (210) and the third electrode layer,
- each of the first and second porous dielectric members (230) is partially immersed in liquid,
- wherein when the voltage is applied to and between the first electrode layer (210) and the second electrode layer (220), and to and between the third electrode layer and the second electrode layer (220), using the voltage source, first dielectric barrier discharge plasma (200) is generated between the first electrode layer (210) and the second electrode layer (220) and second dielectric barrier discharge plasma (200) is generated between the third electrode layer and the second electrode layer (220), and wherein
- the first porous dielectric member (230) is partially located in the first plasma generation region, and the second porous dielectric member (230) is partially located in the second plasma generation region.

7. The plasma generating apparatus of the anion generator according to claim 1, wherein:
- the first electrode (110, 210) is hollow and cylindrical;
- the second electrode (120, 220) is vertical and bar-like and spaced from the first electrode (110, 210), wherein the second electrode (120, 220) is disposed at a central position in the hollow cylindrical first electrode (110, 210);
- the porous dielectric member (130, 230) is hollow and cylindrical and disposed between the first and second electrodes (110, 210; 120, 220), wherein the hollow cylindrical porous dielectric member (130, 230) is spaced from the first and second electrodes (110, 210; 120, 220), wherein the hollow cylindrical porous dielectric member (130, 230) surrounds the vertical bar-like second electrode (120, 220), and wherein
- the voltage source is configured to apply a voltage to and between the first and second electrodes (110, 210; 120, 220).

8. The plasma generating apparatus of the anion generator of claim 6 or 7, further comprising a liquid supply (260) containing therein the liquid, wherein one end of the porous dielectric member (230) is immersed in the liquid in the liquid supply (260) such that the porous dielectric member (230) is impregnated with the liquid.

9. The plasma generating apparatus of the anion generator of any one of claims 1, 6 and 7, wherein the liquid includes hydrogen peroxide.

## Patentansprüche

1. Anionenerzeuger, der eine Plasmaerzeugungseinrichtung umfasst, wobei die Plasmaerzeugungseinrichtung Folgendes umfasst:
- eine erste Elektrode (110);
- eine zweite Elektrode (120), die von der ersten Elektrode (110) beabstandet ist;
- mindestens ein poröses dielektrisches Bauteil (130), das zwischen der ersten und der zweiten Elektrode (110, 120) angeordnet ist,
- eine dielektrische Schicht (150), die auf einer ersten Fläche der ersten Elektrode (110) und/oder der zweiten Elektrode (120) gebildet ist; und
- eine Spannungsquelle (140), die konfiguriert ist, eine Wechselspannung an und zwischen der ersten Elektrode (110) und der zweiten Elektrode (120) anzulegen, wobei, wenn eine Spannung an und zwischen der ersten Elektrode (110) und der zweiten Elektrode (120) unter Verwendung der Spannungsquelle (140) angelegt wird, dielektrisches Sperrentladungsplasma (200) zwischen der ersten Elektrode (110) und der zweiten Elektrode (120) erzeugt wird,
**dadurch gekennzeichnet, dass**:
- das mindestens eine poröse dielektrische Bauteil (130) teilweise in Flüssigkeit eingetaucht ist, und dadurch, dass
- das mindestens eine poröse dielektrische Bauteil (130) teilweise in dem Plasmaerzeugungsbereich liegt.

2. Plasmaerzeugungseinrichtung des Anionenerzeugers nach Anspruch 1, weiter umfassend eine Flüssigkeitsversorgung (160), die die Flüssigkeit darin enthält, wobei ein Ende des porösen dielektrischen Bauteils (130) in die Flüssigkeit in der Flüssigkeitsversorgung (160) eingetaucht ist, so dass das poröse dielektrische Bauteil (130) mit der Flüssigkeit imprägniert wird.

3. Plasmaerzeugungseinrichtung des Anionenerzeugers nach Anspruch 1, wobei sowohl die erste als auch zweite Elektrode (110, 120) sich horizontal erstreckt, wobei das poröse dielektrische Bauteil (130) senkrecht zu der ersten und der zweiten Elektrode (110, 120) ausgerichtet ist, wobei ein erstes Ende des porösen dielektrischen Bauteils (130) die dielektrische Schicht (150) kontaktiert, wobei das erste Ende des porösen dielektrischen Bauteils teilweise in dem Plasmaerzeugungsbereich liegt.

4. Plasmaerzeugungseinrichtung des Anionenerzeugers nach Anspruch 1, wobei sowohl die erste als auch zweite Elektrode (110, 120) sich horizontal erstreckt, wobei das poröse dielektrische Bauteil (130) senkrecht zu der ersten und der zweiten Elektrode (110, 120) ausgerichtet ist, wobei ein erstes Ende des porösen dielektrischen Bauteils (130) von der dielektrischen Schicht (150) abgewandt ist, wobei das erste Ende des porösen dielektrischen Bauteils teilweise in dem Plasmaerzeugungsbereich liegt.

5. Plasmaerzeugungseinrichtung des Anionenerzeugers nach einem der vorstehenden Ansprüche, wobei:
- die erste und zweite Elektrode (110, 120) plattenähnlich sind und wobei
- die zweite Elektrode (120) von der ersten Elektrode (110) abgewandt ist.

6. Plasmaerzeugungseinrichtung des Anionenerzeugers nach Anspruch 5, wobei:
- die plattenähnliche erste Elektrode (110) eine Elektrodenschicht (210) ist;
- die plattenähnliche zweite Elektrode (120) eine Elektrodenschicht (220) ist, die von der ersten Elektrodenschicht (210) beabstandet und parallel dazu ist;
- die Plasmaerzeugungseinrichtung des Anionenerzeugers eine plattenförmige dritte Elektrodenschicht (211) umfasst, die von der zweiten Elektrodenschicht (220) beabstandet und parallel dazu ist, wobei die zweite Elektrodenschicht (220) zwischen der ersten Elektrodenschicht (210) und der dritten Elektrodenschicht angeordnet ist;
- das mindestens eine poröse dielektrische Bauteil ein erstes und ein zweites poröses dielektrisches Bauteil (230) zwischen der ersten Elektrodenschicht (210) und der zweiten Elektrodenschicht (220) beziehungsweise zwischen der zweiten Elektrodenschicht (220) und der dritten Elektrodenschicht angeordnet umfasst, wobei das erste poröse dielektrische Bauteil (230) sowohl von der ersten Elektrodenschicht (210) als auch der zweiten Elektrodenschicht (220) beabstandet ist, wobei das zweite poröse dielektrische Bauteil (230) von sowohl der zweiten Elektrodenschicht (220) als auch der dritten Elektrodenschicht beabstandet ist,
- die Spannungsquelle konfiguriert ist, eine Spannung an und zwischen der ersten Elektrodenschicht (210) und der zweiten Elektrodenschicht (220) und an und zwischen der ersten Elektrodenschicht (210) und der dritten Elektrodenschicht anzulegen,
- sowohl das erste als auch zweite poröse dielektrische Bauteil (230) teilweise in Flüssigkeit eingetaucht ist,
- wobei, wenn die Spannung an und zwischen der ersten Elektrodenschicht (210) und der zweiten Elektrodenschicht (220) und an und zwischen der dritten Elektrodenschicht und der zweiten Elektrodenschicht (220) unter Verwendung der Spannungsquelle angelegt ist, erstes dielektrisches Sperrentladungsplasma (200) zwischen der ersten Elektrodenschicht (210) und der zweiten Elektrodenschicht (220) erzeugt wird und zweites dielektrisches Sperrentladungsplasma (200) zwischen der dritten Elektrodenschicht und der zweiten Elektrodenschicht (220) erzeugt wird, und wobei
- das erste poröse dielektrische Bauteil (230) teilweise in dem ersten Plasmaerzeugungsbereich liegt und das zweite poröse dielektrische Bauteil (230) teilweise in dem zweiten Plasmaerzeugungsbereich liegt.

7. Plasmaerzeugungseinrichtung des Anionenerzeugers nach Anspruch 1, wobei:
- die erste Elektrode (110, 210) hohl und zylindrisch ist;
- die zweite Elektrode (120, 220) vertikal und stabähnlich und von der ersten Elektrode (110, 210) beabstandet ist, wobei die zweite Elektrode (120, 220) bei einer Mittelposition in der hohlen zylindrischen ersten Elektrode (110, 210) angeordnet ist;
- das poröse dielektrische Bauteil (130, 230) hohl und zylindrisch und zwischen der ersten und der zweiten Elektrode (110, 210; 120, 220) angeordnet ist, wobei das hohle zylindrische poröse dielektrische Bauteil (130, 230) von der ersten und der zweiten Elektrode (110, 210; 120, 220) beabstandet ist, wobei das hohle zylindrische poröse dielektrische Bauteil (130, 230) die vertikale stabähnliche zweite Elektrode (120, 220) umgibt, und wobei
- die Spannungsquelle konfiguriert ist, eine Spannung an und zwischen der ersten und der zweiten Elektrode (110, 210; 120, 220) anzulegen.

8. Plasmaerzeugungseinrichtung des Anionenerzeugers nach Anspruch 6 oder 7, weiter umfassend eine Flüssigkeitsversorgung (260), die die Flüssigkeit darin enthält, wobei ein Ende des porösen dielektrischen Bauteils (230) in die Flüssigkeit in der Flüssigkeitsversorgung (260) eingetaucht ist, sodass das poröse dielektrische Bauteil (230) mit der Flüssigkeit imprägniert wird.

9. Plasmaerzeugungseinrichtung des Anionenerzeugers nach einem der Ansprüche 1, 6 und 7, wobei die Flüssigkeit Wasserstoffperoxid beinhaltet.

## Revendications

1. Générateur d'anions comprenant un appareil de génération de plasma, l'appareil de génération de plasma comprenant :
- une première électrode (110) ;
- une seconde électrode (120) espacée de la première électrode (110) ;
- au moins un élément diélectrique poreux (130) disposé entre les première et seconde électrodes (110, 120),
- une couche diélectrique (150) formée sur une première face de la première électrode (110) et/ou de la seconde électrode (120) ; et
- une source de tension (140) configurée pour appliquer une tension alternative aux première électrode (110) et seconde électrode (120), et entre celles-ci,
dans lequel, lorsqu'une tension est appliquée aux première électrode (110) et seconde électrode (120), et entre celles-ci, en utilisant la source de tension (140), un plasma de décharge formant barrière diélectrique (200) est généré entre la première électrode (110) et la seconde électrode (120),
**caractérisé en ce que**
- le au moins un élément diélectrique poreux (130) est partiellement immergé dans un liquide, et **en ce que**
- le au moins un élément diélectrique poreux (130) est partiellement situé dans la région de génération de plasma.

2. Appareil de génération de plasma du générateur d'anions selon la revendication 1, comprenant en outre une alimentation en liquide (160) contenant le liquide, dans lequel une extrémité de l'élément diélectrique poreux (130) est immergée dans le liquide dans l'alimentation en liquide (160) de telle sorte que l'élément diélectrique poreux (130) est imprégné du liquide.

3. Appareil de génération de plasma du générateur d'anions selon la revendication 1, dans lequel chacune des première et seconde électrodes (110, 120) s'étend horizontalement, dans lequel l'élément diélectrique poreux (130) est orienté perpendiculairement aux première et seconde électrodes (110, 120), dans lequel une première extrémité de l'élément diélectrique poreux (130) est en contact avec la couche diélectrique (150), dans lequel la première extrémité de l'élément diélectrique poreux est partiellement située dans la région de génération de plasma.

4. Appareil de génération de plasma du générateur d'anions selon la revendication 1, dans lequel chacune des première et seconde électrodes (110, 120) s'étend horizontalement, dans lequel l'élément diélectrique poreux (130) est orienté perpendiculairement aux première et seconde électrodes (110, 120), dans lequel une première extrémité de l'élément diélectrique poreux (130) fait face à l'opposé de la couche diélectrique (150), dans lequel la première extrémité de l'élément diélectrique poreux est partiellement située dans la région de génération de plasma.

5. Appareil de génération de plasma du générateur d'anions selon l'une quelconque des revendications précédentes, dans lequel :
- les première et seconde électrodes (110, 120) sont en forme de plaque et dans lequel
- la seconde électrode (120) fait face à l'opposé de la première électrode (110).

6. Appareil de génération de plasma du générateur d'anions selon la revendication 5, dans lequel :
- la première électrode de type plaque (110) est une couche d'électrode (210) ;
- la seconde électrode de type plaque (120) est une couche d'électrode (220) qui est espacée de la première couche d'électrode (210), et parallèle à celle-ci ;
- l'appareil de génération de plasma du générateur d'anions comprend une troisième couche d'électrode en forme de plaque (211) espacée de la deuxième couche d'électrode (220), et parallèle à celle-ci, dans lequel la deuxième couche d'électrode (220) est disposée entre la première couche d'électrode (210) et la troisième couche d'électrode ;
- le au moins un élément diélectrique poreux comprend des premier et second éléments diélectriques poreux (230) disposés respectivement entre la première couche d'électrode (210) et la deuxième couche d'électrode (220) et entre la deuxième couche d'électrode (220) et la troisième couche d'électrode, dans lequel le premier élément diélectrique poreux (230) est espacé de chacune de la première couche d'électrode (210) et de la deuxième couche d'électrode (220), dans lequel le second élément diélectrique poreux (230) est espacé de chacune de la deuxième couche d'électrode (220) et de la troisième couche d'électrode,
- la source de tension est configurée pour appliquer une tension aux première couche d'électrode (210) et deuxième couche d'électrode (220), et entre celles-ci, et aux première couche d'électrode (210) et troisième couche d'électrode, et entre celles-ci,
- chacun des premier et second éléments diélectriques poreux (230) est partiellement immergé dans un liquide,
- dans lequel, lorsque la tension est appliquée aux première couche d'électrode (210) et deuxième couche d'électrode (220), et entre celles-ci, et aux troisième couche d'électrode et deuxième couche d'électrode (220), et entre celles-ci, en utilisant la source de tension, un premier plasma de décharge formant barrière diélectrique (200) est généré entre la première couche d'électrode (210) et la deuxième couche d'électrode (220) et un second plasma de décharge formant barrière diélectrique (200) est généré entre la troisième couche d'électrode et la deuxième couche d'électrode (220), et dans lequel
- le premier élément diélectrique poreux (230) est partiellement situé dans la première région de génération de plasma, et le second élément diélectrique poreux (230) est partiellement situé dans la seconde région de génération de plasma.

7. Appareil de génération de plasma du générateur d'anions selon la revendication 1, dans lequel :
- la première électrode (110, 210) est creuse et cylindrique ;
- la seconde électrode (120, 220) est verticale et de type barre et espacée de la première électrode (110, 210), dans lequel la seconde électrode (120, 220) est disposée à une position centrale dans la première électrode cylindrique creuse (110, 210) ;
- l'élément diélectrique poreux (130, 230) est creux et cylindrique et disposé entre les première et seconde électrodes (110, 210; 120, 220), dans lequel l'élément diélectrique poreux cylindrique creux (130, 230) est espacé des première et seconde électrodes (110, 210; 120, 220), dans lequel l'élément diélectrique poreux cylindrique creux (130, 230) entoure la seconde électrode verticale de type barre (120, 220), et dans lequel
- la source de tension est configurée pour appliquer une tension aux première et seconde électrodes (110, 210 ; 120, 220), et entre celles-ci.

8. Appareil de génération de plasma du générateur d'anions selon la revendication 6 ou 7, comprenant en outre une alimentation en liquide (260) contenant le liquide, dans lequel une extrémité de l'élément diélectrique poreux (230) est immergée dans le liquide dans l'alimentation en liquide (260) de telle sorte que l'élément diélectrique poreux (230) est imprégné du liquide.

9. Appareil de génération de plasma du générateur d'anions selon l'une quelconque des revendications 1, 6 et 7, dans lequel le liquide inclut du peroxyde d'hydrogène.
